(19) **Europäisches Patentamt**
**European Patent Office**
**Office européen des brevets**

(11) **EP 2 082 686 B1**

(12) **EUROPÄISCHE PATENTSCHRIFT**

(45) Veröffentlichungstag und Bekanntmachung des Hinweises auf die Patenterteilung:
**21.01.2015 Patentblatt 2015/04**

(51) Int Cl.:
***A61B 6/00*** *(2006.01)*

(21) Anmeldenummer: **08150480.5**

(22) Anmeldetag: **22.01.2008**

(54) **Orientierte Wiedergabe von Aufnahmen**

Method to display orientated (overlap) images

Retransmission de réception orientée

(84) Benannte Vertragsstaaten:
**DE FR GB**

(43) Veröffentlichungstag der Anmeldung:
**29.07.2009 Patentblatt 2009/31**

(73) Patentinhaber: **Brainlab AG**
**85622 Feldkirchen (DE)**

(72) Erfinder:
• **Uhde, Jörg**
**80538, München (DE)**

• **Bogojevic, Alejksander**
**81677 München (DE)**

(74) Vertreter: **Schwabe - Sandmair - Marx**
**Patentanwälte**
**Stuntzstraße 16**
**81677 München (DE)**

(56) Entgegenhaltungen:
**EP-A- 1 782 734 DE-A1- 10 015 815**
**DE-A1- 10 210 645 DE-A1- 10 322 739**
**DE-A1- 10 360 025 US-A1- 2003 179 856**

EP 2 082 686 B1

**EP 2 082 686 B1**

**Beschreibung**

Die Erfindung betrifft ein Verfahren zur orientierten Darstellung von Aufnahmen von einem in wenigstens zwei Aufnahmeschritten aufgenommenen, abzubildenden Bereich, gemäß dem Anspruch 1.

Insbesondere wird ein Verfahren vorgeschlagen, um von einem Körperbereich eines Patienten mittels Röntgen erstellte Aufnahmen zum Zwecke der computergestützten Navigation zueinander zu orientieren.

Um aussagekräftige Bilder im Hinblick auf Körperteile eines Menschen oder Tieres beispielsweise für die Orthopädie oder die Traumatologie zu erhalten, ist es üblich, mit einem Röntgengerät beispielsweise zwei Aufnahmen eines Körperbereiches, d.h., etwa einer anatomischen Struktur, zu machen.

Die wenigstens zwei Röntgenaufnahmen werden durch eine örtliche Verlagerung des Röntgengerätes relativ zu dem aufzunehmenden Bereich des Patienten erhalten.

Üblicherweise werden dabei mehrere Aufnahmen auf zwei getrennten Monitoren abgebildet. Eine ganz übliche Konfiguration ist ein mobiles Röntgengerät, auch als "C-Bogen" bezeichnet, in Verbindung mit zwei Monitoren. Dabei wird auf einem Monitor ein aktuelles und auf einem weiteren Monitor ein beliebiges vorher aufgenommenes Bild des interessierenden Körperbereichs angezeigt, welches jederzeit durch das aktuelle Bild ersetzt werden kann. Problematisch ist hierbei, dass die Bilder keine definierte Orientierung, keine maßstäbliche Beziehung oder dergleichen, zueinander haben. D.h., die dargestellten Bilder können zueinander in sämtlichen Raumrichtungen verkippt sein, und der Anschluss der Bilder aneinander sowie die Darstellung mit dem gleichen Maßstab sind systematisch nicht möglich.

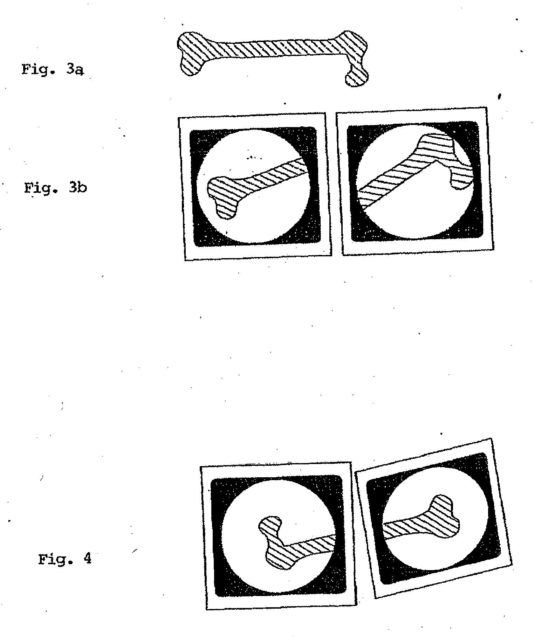

In der Fig. 3b ist eine anatomische Struktur wiedergegeben, hier ein Hüftknochen, der mit einem C-Bogen mit zwei Aufnahmen an verschiedenen Positionen entlang des Körpers eines Patienten aufgenommen worden ist. Diese zwei Aufnahmen oder Röntgenbilder der anatomischen Struktur werden auf zwei Monitoren des C-Bogens dargestellt. Wie zu erkennen ist, haben die auf den verschiedenen Monitoren dargestellten Abschnitte der anatomischen Struktur gemäß Fig. 3a, wie aus Fig. 3b ersichtlich, unterschiedliche Ausrichtungen und Abbildungsvergrößerungen. Eine Orientierung anhand dieser Bildschirminhalte gemäß Fig. 3b ist reproduzierbar und realitätsentsprechend nicht möglich.

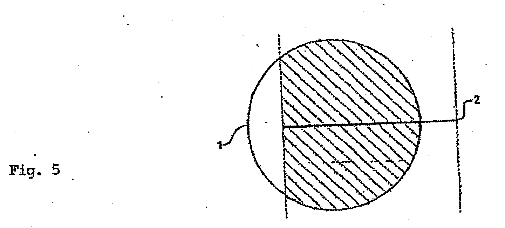

Eine weitere Zielsetzung gegenüber dem Stand der Technik kann darin gesehen werden, sich dreidimensional in dem Körper oder an dem Körper eines Patienten zurechtzufinden, etwa um eine Sonde zu setzen oder um eine anatomische Struktur zu behandeln. Hierzu ist es nötig, dass zwei oder mehr möglichst genau überlappende Röntgenaufnahmen für eine Navigation erstellt werden, deren Überlappungsgeometrie in definierbarer Weise bekannt sein sollte. Die Zentralstrahlen dieser Röntgenaufnahmen sollten einen möglichst exakten Schnittpunkt aufweisen. Die Anordnung der mehreren überlappenden Röntgenaufnahmen führt zu einer Lageinformation in drei Dimensionen, wobei die nötigen Informationen für eine Darstellung im Raum bei einer Minimierung der Abbildungsfehler aus zwei Röntgenaufnahmen zu entnehmen sind, die möglichst senkrecht zueinander aufgenommen worden sind. Dadurch, dass die Röntgenaufnahmen einander überlappen müssen, wird der effektive Abbildungsbereich, in dem mit voller dreidimensionaler Information navigiert werden kann, verkleinert. Diesbezüglich sei auf Fig. 5 verwiesen, in der ein Durchleuchtungsbild 1 dargestellt ist, das in der Ebene des Papierblattes der Fig. 5 liegt. Ein weiterer Durchleuchtungsbildbereich 2 liegt senkrecht zum Durchleuchtungsbild 1, wobei keine genaue Überlappung erkennbar ist. Der sich aus diesen beiden Bildern 1, 2 für eine Navigation ergebende verwendbare Bereich ist schraffiert dargestellt. Wie zu erkennen ist, ist der verwertbaren Bilddurchmesser aufgrund von zwei Durchleuchtungsbildern im besten Fall so groß wie der Durchmesser eines Durchleuchtungsbildes und in der Regel noch kleiner.

EP 1 782 734 bezieht sich auf ein Verfahren zur Verbesserung der Volumenrekonstruktion bei navigationsgeführten Eingriffen, denen 2D-Röntgenprojektionsaumahmen zugrunde gelegt werden. Es wird ein Phantom mit Markern, die sowohl auf einem Röntgenbild erkennbar als auch vom Navigationssystem erfassbar ist, verwendet. Die Aufgabe einer Erfindung ist es, die Ermittlung einer Abbildungsvorschrift von mit einem Röntgengerät aufgezeichneten 2D-Projektionen des Phantoms zu einem 3D-Volumendatensatz, in dem ein Instrument gerührt wird aufzuzeigen. Wesentlich ist es, dass das mit Röntgenpositiven und vom Navigationssystem erfassbaren Markern ausgestattete Phantom mit dem Untersuchungsobjekt, d.h. mit dem Patientenkörper verbunden bleibt.

US 2003/0179856 offenbart eine Vorrichtung zur Bestimmung einer Koordinatentransformation durch Mischen eines Röntgenbildes eines ersten Objekts mit einem Röntgenbild eines zweiten Objekts. Es wird eine Markerhalterung verwendet, um röntgenpositive Marker und vom Navigationssystem erfassbare Marker am Patientenkörper zu befestigen. Die Koordinaten der röntgenpositiven Marker werden in einem Koordinatensystem, das dem Navigationssystem zugewiesen ist, erfasst. Ziel der Erfindung ist es, eine Transformation zwischen dem dem Navigationssystem zugewiesenen Koordinatensystem und einem dem Röntgenbild zugewiesenen Koordinatensystem zu ermitteln.

DE 10 210 645 offenbart ein Verfahren zur Erfassung und Darstellung eines in einen Untersuchungsbereich eines Patienten eingeführten medizinischen Katheters. Das betreffende Instrument soll in einer dreidimensionalen Darstellung des Untersuchungsbereichs positionsrichtig im Volumenbild dargestellt werden. Zu diesem Zweck wird eine geeignete Transformationsmatrix genutzt, um die Koordinaten der Katheterspitze aus dem Koordinatensystem eines Navigationssystems in das eines 3 D-Rekonstruktionsbilds zu transformieren. Hierbei werden im 3D-Rekonstruktionsbild und im Koordinatensystem des Navigationssystems definierte Markierungen verwendet, wobei derartige Markierungen durch Bewegen des Katheters an die Markierungspositionen definiert werden.

Es ist die Aufgabe gemäß der vorliegenden Erfindung, die orientierte Wiedergabe von Aufnahmen von einem in wenigstens zwei Aufnahmeschritten aufgenommenen, abzubildenden Bereich auf wenigstens einem Bildschirm zu ermöglichen. Insbesondere soll es ermöglicht werden, mit mehreren Aufnahmen aufgenommene Bilder systematisch aneinander anzupassen bzw. orientiert aneinander anzusetzen oder zusammenzusetzen und insbesondere weiterhin diese zueinander orientierten oder orientiert zusammenzusetzen Aufnahmen für die Navigation zu verwenden.

Die gemäß der Erfindung erzielbaren Vorteile beruhen auf dem Verfahren gemäß dem Patentanspruch 1. Zweckmäßige Verfahrensvarianten des erfindungsgemäßen Verfahrens sind den Unteransprüchen zu entnehmen.

Natürlich gehören zur Erfindung auch Programme, die das erfindungsgemäße Verfahren realisieren sowie Datenträger, die entsprechende Programme zur Ausführung auf einen Computer zur Verwirklichung des erfindungsgemäßen Verfahrens in digitaler Form speichern.

Nachfolgend wird die vorliegende Erfindung anhand einer bevorzugten Ausführungsform unter Bezugnahme auf die beigefügten Darstellungen näher beschrieben, wobei weitere Merkmale, Vorzüge sowie Zielsetzungen gemäß der Erfindung offenbart werden. In den Darstellungen zeigen:

| | |
|---|---|
| Fig. 1a und 3a | eine anatomische Struktur in ihrer ursprünglichen Form |
| Fig. 1b und 4 | eine anatomische Struktur, die nicht überlappend aufgenomen wurde, jedoch nach dem erfindungsgemäßen Verfahren orientiert und skaliert ist |
| Fig. 2 | eine Aufnahmeanordnung zur Durchführung des erfindungsgemäßen Verfahren |
| Fig. 3b | eine anatomische Struktur, die nicht überlappend aufgenomen wurde, in zwei nicht orientierten Darstellungen gemäß dem Stand der Technik |
| Fig. 5 | Durchleuchtungsbilder in der Ebene des Papierblattes und senkrecht dazu |

Vorteilhafterweise werden wenigstens zwei Aufnahmen in wenigstens näherungsweise derselben Ebene erzeugt. Durch die Aufnahme von zwei Durchleuchtungs- bzw. Röntgenbildern in wenigstens näherungsweise derselben Ebene kann es ermöglicht werden, eine Ähnlichkeit der Positionen der Röntgenquelle bei den Aufnahmen herzustellen.

Begleitet wird diese Vorgehensweise von dem Einsatz eines Navigationssystems mit einem Stereo-Aufnahmesystem, in der Regel einer Stereo-Kamera. Dabei sind z.B. Marker am Röntgendetektor und am abzubildenden Bereich in vorgegebener Weise angeordnet und können vom Navigationssystem etwa unter Verwendung einer Kalibrierung Raumkoordinate zugeordnet werden. Wird eine Aufnahme gemacht, wird gleichzeitig mit dieser Röntgenaufnahme das Stereoaufnahmesystem in Aktion gesetzt, um Daten über die räumliche Beziehung der Röntgenaufnahme zum abzubildenden Bereich zu erhalten. Das Navigationssystem erfasst und speichert die aktuellen Positionen der Röntgeneinheit und der am abzubildenden Bereich vorgesehenen Marker, die vom Navigationssystem erkannt werden. Durch eine anschließende Datenverarbeitung wird aus der Position des Detektors im Raum und aus der verzerrten Anordnung von einigen erkannten Markern, deren reale Position zueinander bekannt ist, die Position der Röntgenquelle und die Verzerrung der Abbildung des abzubildenden Bereichs ermittelt. Anschließend wird jeder Position im Koordinatensystem des abzubildenden Bereichs bzw. des anatomischen Objekts ein projizierter Punkt des angezeigten Röntgenbildes zugewiesen. Die Projektionslinien verlaufen dabei von der relativen Position aus, welche die Röntgenquelle zum Zeitpunkt der Aufnahme im Verhältnis zum abzubildenden Bereich hat.

Dabei ist es von Vorteil, wenn ein auswählbarer Ausschnitt des Aufnahmebereichs also eine Einzelaufnahme, parallel zur Bildschirmoberfläche wiedergegeben wird. D.h., ein ausgewählter Bereich wird in die Bildschirmoberfläche gedreht bzw. transformiert, um parallel dargestellt zu werden, während andere, zu dem ausgewählten Ausschnitt benachbarte Ausschnitte ihrer Orientierung entsprechend in bzw. aus der Bildschirmoberfläche hinein- bzw. herauslaufend wiedergegeben werden. Um dem Betrachter die Orientierung der verschiedenen Bildausschnitte zueinander besser sichtbar zu machen, können die Bereiche, die benachbart zu dem ausgewählten Ausschnitt sind, abgesetzt durch einen erkennbaren Rand oder Ränder und/oder eine flächenhafte Markierung, wie etwa eine Schraffur, eine Schattierung oder dgl., auf dem Bildschirm deutlich erkennbar gemacht werden.

Damit können mittels der vorliegenden Erfindung in einer bevorzugten Ausführungsform gleichzeitig zwei oder mehrere Durchleuchtungsbilder in korrekter Orientierung zueinander maßstabsgetreu dargestellt werden.

Eine Anpassung der registrierten Röntgenaufnahmen aneinander, bzw. der ersten Röntgenaufnahme, die als Referenzbild dienen kann, an die zweite Röntgenaufnahme kann mit nachfolgendem Algorithmus bzw. Verfahren durchgeführt werden, wobei dieses lediglich als Beispiel anzusehen ist. Natürlich gibt es andere Näherungsalgorithmen, die dazu führen können, dass das zweite Röntgenbild in seiner Größe, Entfernung, Rotation und Verkippung passend zum ersten Röntgenbild bzw. zum Referenzbild auf einem oder mehreren Bildschirmen dargestellt werden kann.

Röntgenbilder, als eine Art Schattenriss ohne Tiefeninformation, können ohne Beschränkung der Allgemeinheit als im Bildverstärker liegend, im 3D-Raum angenommen werden:

3

$$\mathbf{R}^i(x_B^i) = E_B^i$$

Dabei sind die $x_B^i$ Raumpunkte im Koordinatensystem des Bildverstärkers, die $E_B^i$ Mögliche (zum Mittelstrahl senkrechte) Schnittebenen durch den Strahlkegel, welche die entzerrte Bildinformation des Röntgenbildes enthalten und welche an der Stelle des Bildverstärkers liegen und **R e**ine Abbildungsvorschrift die beschreibt, an welcher Stelle von $E_B^i$ ein Raumpunkt $x_B^i$ vom Röntgenstahlkegel abgebildet wird.

Es gibt nun eine oder mehrere Koordinatensystemtransformationen $\mathbf{T}_W(B^i,B^j)$, welche die Änderung der dreidimensionalen Lage des Bildverstärkers von der Position zum Zeitpunkt der Aufnahme *i* eines Bildes, zur Position zum Zeitpunkt der Aufnahme eines weiteren Bildes *j*, in einem Koordinatensystem der Welt *W* beschreiben (dies ist z.B. das System einer ortsfesten Kamera oder üblicherweise das Referenzstemsystem, welches mit dem abzubildenden Objekt verbunden ist):

$$E_W^j = \mathbf{T}_W(B^i, B^j) \cdot E_W^j$$

Dabei sind die $E_W^j$, $E_W^j$ die 3D-Bildebenen zu verschiedenen Röntgenaufnahmen im gemeinsamen Koordiantensystem der Welt. Die Transformation $\mathbf{T}_W(B^i,B^j)$ kann zum Beispiel aus den Transformationen der Position des Bildwandlers zu den Zeitpunkten der Aufnahmen $B_W^i$, $B_W^j$ in den Ursprung des Weltkoordiantensystems zusammengesetzt werden:

$$\mathbf{T}_W(B^i, B^j) = (\mathbf{T}_{B_W^j \to 0_W})^{-1} \cdot \mathbf{T}_{B_W^i \to 0_W} \cdot$$

Die oben beschrieben Transformationen können zum Beispiel klassische 3x4-Translations-Rotations-Matrizen oder zum Beispiel auch die in der Computergrafik übliche 4x4-Matritzen mit zusätzlichen Skalierinformationen sein.

Wird das eine Bild (nach Entzerrung und Registrierung) als Textur einer Ebene in Koordinaten des Computergrafiksystems dargestellt (z.B. im Vertex-Koordinatensystem von openGL), kann die gleiche Transformation $\mathbf{T}_W(B^i, B^j)$ benutzt werden, um das zweite Bild im Sinne der Erfindung zu orientieren und mittels des Computergrafiksystems skaliert und orientiert zum ersten Bild darzustellen:

$$E_V^j = \mathbf{T}_V(B^i, B^j) \cdot E_V^j$$

Zweckmäßigerweise wird dazu eine (zum Beispiel die erste) Ebene parallel zum Monitor orientiert dargestellt und initial so skaliert, dass mehrere oder alle anzuzeigenden Aufnahmen auf dem Bildschirm in korrekter Skalierung und Entfernung dargestellt werden können. Dazu wird zum Beispiel der Abstand zum nächsten Bild in Einheiten der Bildbreite eines Bildes berechnet und die Skalierung folgendermaßen gewählt:

Skalierung = (Maximale am Bildschirm darstellbare Breite in Pixeln) / (Maximale Bildbreite in Einheit X + maximaler Zentralabstand zu einem weiteren Bild in Einheit X).

Um weitere Bild- bzw. Orientierungsinformationen für einen Operateur bzw. Arzt bereit zu stellen wird nach der Fertigung einer ersten und einer zweiten Aufnahme gemäß der Erfindung das Röntgengerät in eine andere Stellung zu verfahren, um dort in einem anderen Winkel zu dem aufzunehmenden Bereich wiederum wenigstens zwei Aufnahmen nach dem erfindungsgemäßen Verfahren aufzunehmen. Dabei ist es besonders vorteilhaft, wenn der zweite Satz einer ersten und einer zweiten Aufnahme in einem bestimmten Winkel, bevorzugt 90°, zum ersten Satz einer ersten und zweiten Aufnahme erzielt worden ist, da derart optimierte räumliche Bildinformationen erhalten werden können. Die jeweils überlappenden Aufnahmen der beiden voneinander eventuell distanzierten und/oder zueinander verwinkelt aufgenommenen Sätze von Aufnahmen können dann zueinander gemäß der Erfindung registriert und orientiert werden, um zu einem zusammenhängenden Bild auf einem oder mehreren Bildschirmen zusammengesetzt zu werden. Dabei werden mittels der Stereokamera und den von dieser erfassten Markern räumlichen Zuordnungen zwischen den Bildinformationen der Röntgenaufnahmen rechnerisch erstellt.

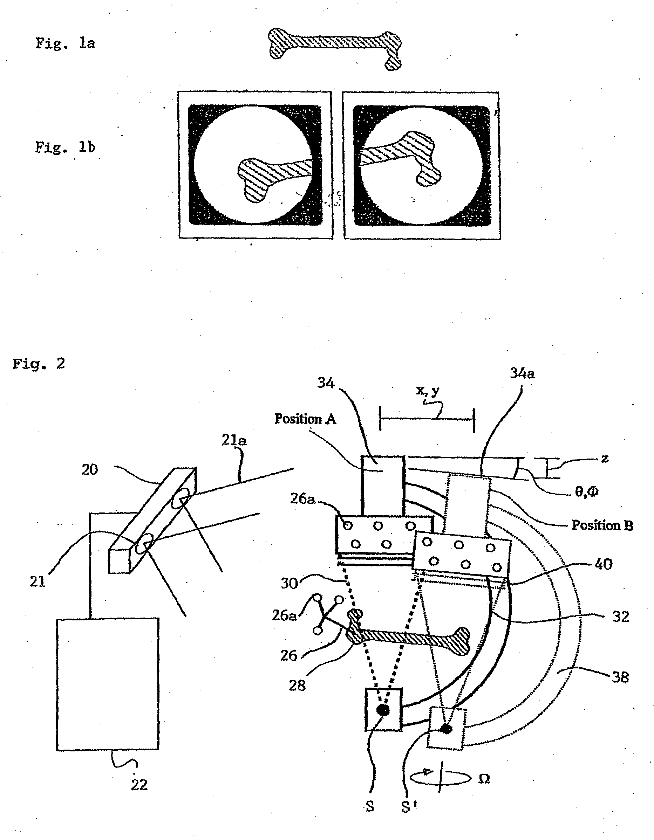

Die Fig. 1a und 3a zeigen eine anatomische Struktur in der Form eines menschlichen Hüftknochens. Es gilt nun, diese anatomische Struktur mittels eines Röntgengerätes, insbesondere eines sog. C-Bogens, aufzunehmen und die dabei erzeugten Durchleuchtungsbilder so wiederzugeben, dass es dem Betrachter ermöglicht wird, aus den bereitgestellten Bildinformationen den größtmöglichen Nutzen zu ziehen. D.h., die dargestellten Bildinhalte müssen genau sein, zueinander passen sowie nach Möglichkeit in Bezug auf ihre Abmessungen, Dimensionen, Verwinklungen, usw. aneinander angepasst sein oder wenigstens erkennbar gemacht sein.

Werden die Röntgenaufnahmen beispielsweise in zwei Anzeigebereichen wiedergegeben, kann, wie in Fig. 4 gezeigt, eine Registrierung der beiden Bildinhalte der beiden Aufnahmen, die das Röntgengerät gemacht hat, vorgenommen werden, anhand derer sich der Betrachter problemlos orientieren kann. Ein Vergleich mit der Fig. 3b macht den Unterschied zum Stand der Technik sofort deutlich. Während gemäß der Erfindung eine registrierte Darstellung der Bildinhalte erfolgt, wobei die Bildinhalte bezüglich ihrer Abmessungen, Vergrößerungen, etc. zueinander passen, kann gemäß dem Stand der Technik von einer genauen Orientierung anhand der Abbildungen keine Rede sein (siehe Fig. 3a, 3b, 4).

In der Fig. 2 wird der prinzipielle Aufbau eines navigierten Röntgenaufnahmesystems dargestellt, wie er zur Ausführung des Verfahrens nach der vorliegenden Erfindung zum Einsatz gelangen könnte. In der Fig. 2 ist ein Navigationssystem prinzipiell dargestellt, das unter anderem eine Stereo-Kamera 20 mit einem Paar von Objektiven 21 umfasst. Die Stereo-Kamera ist an eine Datenverarbeitungsanlage 22 angeschlossen. Die Objektive 21 der Stereo-Kamera, beispielsweise einer Infrarot-Stereo-Kamera, weisen jeweilige Erfassungswinkel 21a bzw. entsprechende Erfassungsbereiche auf, innerhalb derer die Existenz von Markern 26a erfasst und positionsmäßig verarbeitet werden kann.

An einem Röntgendetektor 34, 34a sind Marker 26a vorgesehen. Der Röntgendetektor 34 liegt einer Röntgenquelle S, S' gegenüber. Zwischen dem Röntgendetektor 34 und der Röntgenquelle S, S' erstreckt sich ein Aufnahmekegel 30, 32, in dem ein abzubildendes Objekt 28, hier ein Oberschenkelknochen 28, durch eine, insbesondere zwei bzw. mehre Röntgenaufnahmen erfasst und abgebildet werden soll. An dem Oberschenkelknochen 28 sind ebenfalls mehrere Marker 26a in der Form eines Markerkits 26 mit mehreren Markern 26a positionsfest angeordnet.

An dem Röntgendetektor 34 ist zum Zweck der Kalibrierung eine Kalibrierungsanordnung 40 angeordnet.

Die prinzipielle Funktionsweise eines derartigen Röntgengerätes in Verbindung mit einem Navigationssystem beginnt nach dem erfindungsgemäßen Verfahren damit, eine erste Aufnahme des abzubildenden Bereichs durch das Röntgengerät von einer ersten Position aus zu erstellen, wobei ein erstes Steuersignal abgegeben wird. Andererseits kann die Navigationseinrichtung über eine Bildanalyse das Auslösen der Aufnahme detektiert und ein erstes Steuersignal ausgeben. Durch das erste Steuersignal wird das Stereo-Aufnahmesystem 20, 21 dazu veranlasst, erste Positionen der mehreren Markereinrichtungen 26a am Röntgendetektor und am abzubildenden Bereich zu erfassen.

Die Daten zu den ersten Positionen der Markereinrichtungen 26a werden gespeichert und rechnergestützt in ein Verhältnis zu Bildpunkten der ersten Röntgenaufnahme gesetzt, um die Röntgenaufnahme zu registrieren. Danach wird der Röntgendetektor zusammen mit der Röntgenquelle S, S' relativ zum abzubildenden Bereich in eine zweite Position S' verbracht und durch den Röntgendetektor wird eine zweite Aufnahme ausgelöst, wobei ein zweites Steuersignal abgegeben wird. Mittels des zweiten Steuersignals wird das Stereo-Aufnahmesystem 20, 21 dazu veranlasst, zweite Positionen der Markereinrichtungen 26a am Röntgendetektor und am abzubildenden Bereich zu erfassen.

Danach werden die Daten zu den zweiten Positionen der Markereinrichtungen 26a rechnergestützt in ein Verhältnis zu Bildpunkten der Röntgenaufnahme gesetzt, um die zweite Röntgenaufnahme zu registrieren. Über das gemeinsame Koordinatensystem des abzubildenden Bereichs wird eine Transformationsmatrix für die Lageveränderung des Detektors 34 zwischen der ersten Aufnahme gemäß Position A in Fig. 2 und der zweiten Aufnahme gemäß Position B in Fig. 2 bestimmt. Mittels einer darauf basierenden Matrix werden die Aufnahmen zueinander orientiert und skaliert, wobei aus der Lageveränderung in z-Richtung die maßstabsgerechte Größenskalierung aus einer Lageveränderung in x- und/oder y-Richtung der maßstabsgerechter Abstand der Bilder, aus einem dem Winkel $\Omega$ die maßstabsgerechte Rotation und aus Winkeln $\Theta$ und $\Phi$ die maßstabsgerechte Verkippung der zweiten Aufnahme zur Ebene der ersten Aufnahme ermittelt wird. Nun werden die beiden Aufnahmen auf einem oder mehreren Bildschirmen zueinander orientiert dargestellt, wie sich dies etwa aus Fig. 4 ergibt. Dabei werden vorteilhafterweise werden zwei Aufnahmen in wenigstens näherungsweise derselben Ebene erzeugt.

Gemäß Fig. 2 wird der Röntgendetektor 34, der sich an einem C-Bogen 38 montiert befindet, und demgegenüber die Röntgenquelle S an dem C-Bogen angeordnet ist, beliebig in seiner Position verbracht, um einerseits eine Röntgenaufnahme aus der Position A und andererseits eine Röntgenaufnahme aus der Position B zu erstellen. Die Position von navigierten Werkzeugen kann immer aus den genauen Positionen der Projektionszentren berechnet und auf den dargestellten Bildern angezeigt werden.

Das soeben beschriebene Wiedergabeverfahren funktionieren insbesondere auch dann, wenn sich die Röntgenstrahlkegel nicht überlappen. Der Bereich zwischen den Bildern kann dann z.B. schwarz dargestellt werden. Überlappen sich die Bilder muss in der Darstellung von navigierten Werkzeugen unterschieden werden, auf welche Strahlprojektionen sich die Werkzeugdarstellung beziehen. Zum Beispiel kann ein Werkzeug bei einer Überlappung nur im für den Betrachter vorderen (oder hinteren) Bildteil oder auch doppelt (entlang beider Strahlprojektionen) angezeigt werden. Zweckmäßigerweise kennzeichnet man im letzten Fall die Werkzeugdarstellung so, dass klar ist, auf welches Bild sie sich bezieht (zum Beispiel durch Einfärbung).

Gemäß der Erfindung können auch mehr als zwei Bilder zusammengefasst werden.

Mit den unter Bezugnahmen auf die Figuren 2 und 5 beschriebenen Verfahren lässt sich ein Satz von Aufnahmen mit einer ersten und einer zweiten Röntgenaufnahme erstellen. Weiterhin lässt sich ein zweiter Satz mit einer ersten und einer zweiten Röntgenaufnahme gemäß der Erfindung erstellen. Die allgemeine Orientierung des ersten Satzes kann dann zur allgemeinen Orientierung des zweiten Satzes verwinkelt, insbesondere um 90° und/oder distanziert aufgenommen werden. Natürlich lassen sich noch weitere Sätze von zwei oder mehr Röntgenaufnahmen entsprechend herstellen und zueinander orientiert bzw. registriert werden, um diese überlagert auf einem oder mehreren Bildschirmen wiederzugeben.

Insgesamt lässt sich gemäß der Erfindung und deren Ausführungsformen der insbesondere für eine dreidimensionale Navigation verwendbare Bereich ganz erheblich vergrößern, d.h., insbesondere der Bereich, der aus Fig. 5 hervorgeht.

## Patentansprüche

1.  Verfahren zur orientierten Darstellung von Aufnahmen von einem in wenigstens zwei Aufnahmeschritten mit einem Navigationssystem (20, 21, 22) aufgenommenen, abzubildenden Bereich, insbesondere eines Oberschenkeltrochens (28), wobei die Aufnahmen in einem gemeinsamen Koordinatensystem des abzubildenden Bereichs lokalisierbar sind, wobei das Navigationssystem (20, 21, 22) folgende Merkmale umfasst:

    - einen Röntgendetektor (34, 34a) mit einer diesem gegenüber angeordneten Röntgenquelle (S, S'), die zueinander in einem vorgebbaren Verhältnis gemeinsamen und zu dem abzubildenden Bereich bewegbar sind;
    - einem Stereo-Aufnahmesystem (20), etwa einer Stereokamera;
    - mehreren Markereinrichtungen (26a), die an dem Röntgendetektor (34, 34a) und dem abzubildenden Bereich positionsfest angeordnet sind, und die wenigstens teilweise durch das Stereo-Aufnahmesystem (20) erfassbar sind;

    wobei das Verfahren durch die nachfolgenden Schritte gekennzeichnet ist:

    a) eine erste Aufnahme des abzubildenden Bereichs wird durch das Röntgengerät in einer ersten Position ausgelöst,
    b) die Navigationseinrichtung detektiert über eine Bildanalyse das Auslösen der Aufname und gibt ein erste Steuersignal aus;
    c) durch das erste Steuersignal wird das Stereo-Aufnahmesystem (20) veranlasst, erste Positionen der mehreren Markereinrichtungen (26a) am Röntgendetektor (34) und am abzubildenden Bereich zu erfassen;
    d) Daten zu den ersten Positionen werden gespeichert;
    e) die Daten zu den ersten Positionen werden rechnergestützt in ein Verhältnis zu Bildpunkten der Röntgenaufnahme gesetzt, um die Röntgenaufnahme zu registrieren;
    f) der Röntgendetektor (34, 34a) wird zusammen mit der Röntgenquelle (S, S') relativ zum abzubildenden Bereich in eine zweite Position verbracht;
    g) eine zweite Aufnahme wird durch den Röntgendetektor (34, 34a) ausgelöst, wobei ein zweites Steuersignal abgegeben wird;
    h) durch das zweite Steuersignal wird das Stereo-Aufnahmesystem (20) veranlasst, zweite Positionen der Markereinrichtungen (26a) am Röntgendetektor (34, 34a) und am abzubildenden Bereich zu erfassen;
    i) Daten zu den zweiten Positionen werden rechnergestützt in ein Verhältnis zu Bildpunkten der Röntgenaufnahme gesetzt, um die zweite Röntgenaufnahme zu registrieren;
    j) über das gemeinsame Koordinatensystem des abzubildenden Bereichs wird eine Transformationsmatrix für die Lageveränderung des Röntgenstrahlkegels (30, 32) zwischen der ersten und der zweiten Aufnahme bestimmt;
    k) die Aufnahmen werden mit der Matrix zueinander orientiert und skaliert, wobei aus der Lageveränderung in z-Richtung eine maßstabsgerechte Größenskalierung, aus einer Lageveränderung in x- und/oder y-Richtung ein maßstabsgerechter Abstand der Aufnahmen, aus einem Winkel Q eine maßstabsgerechte Rotation und aus Winkeln Θ und Φ eine maßstabsgerechte Verkippung der zweiten Aufnahme zur Ebene der ersten Aufnahme bestimmt wird;

l) die Aufnahmen werden mit den bestimmten Werten zueinander orientiert und dargestellt.

2. Verfahren nach einem der voranstehenden Ansprüche, wobei weitere Röntgenaufnahmen von anderen Verbringungspositionen her gemacht werden und entsprechend der zweiten Röntgenaufnahme an die erste Röntgenaufnahme oder an eine aufgrund dieser erzeugte Gesamtdarstellung in angepasster Form angegliedert werden.

3. Verfahren nach einem der voranstehenden Ansprüche, wobei ein erster Satz von Aufnahmen, der die erste und die zweite Aufnahme umfasst, relativ zu einer ersten gemeinsamen Position erstellt wird, und ein zweiter Satz von Aufnahmen, der eine weitere erste und zweite Aufnahme enthält, in einer zweiten gemeinsamen Position, die von der ersten verschieden ist, erstellt wird, wobei die gemeinsamen Positionen um einen Winkel zueinander verdreht sind und/oder voneinander distanziert sind.

4. Verfahren nach Anspruch 3, wobei der Winkel in etwa 90° beträgt.

5. Verfahren nach einem der voranstehenden Ansprüche, wobei mögliche Positionierungsmaßnahmen mittels einer Anzeigeeinrichtung einer Bedienungsperson angezeigt werden.

6. Verfahren nach einem der voranstehenden Ansprüche, wobei die Aufnahmeanordnung eine motorische Anordnung umfasst, die die gewünschte Positionierungsmaßnahmen manuell gesteuert oder automatisch umsetzt.

**Claims**

1. A method for oriented display of images of a region to be imaged, in particular of a femoral bone (28), which has been imaged in in at least two imaging steps using a navigation system (20, 21, 22), wherein the images are localizable in a common coordinate system of the region to be imaged, wherein the navigation system (20, 21, 22) comprises the following features:

   - an x-ray detector (34, 34a) having an x-ray source (S, S') disposed opposite to it, which are movable in a presettable relationship relative to each other and relative to the region to be imaged;
   - a stereo-acquisition system (20) such as a stereo camera;
   - a plurality of marker devices (26a) which are disposed positionally fixed on the x-ray detector (34, 34a) and the region to be imaged and which are at least partially detectable by the stereo-acquisition system (20);
   - wherein the method is **characterised by** the following steps:

      a) a first image of the region to be imaged is triggered by the x-ray device at a first position,
      b) the navigation device detects triggering of the imaging via an image analysis and outputs a first control signal;
      c) the stereo-acquisition system (20) is made by the first control signal to acquire first positions of the plural marker devices (26a) on the x-ray detector (34) and the region to be imaged;
      d) data about the first positions is saved;
      e) the data about the first positions is related, using a computer, to image points of the x-ray image in order to register the x-ray image;
      f) the x-ray detector (34, 34a) is moved, relative to the region to be imaged and together with the x-ray source (S, S'), into a second position;
      g) a second image is triggered by the x-ray detector (34, 34a), outputting a second control signal;
      h) the stereo-acquisition system (20) is made, by the second control signal, to acquire second positions of the marker devices (26a) on the x-ray detector (34, 34a) and on the region to be imaged;
      i) data about the second positions is related, using a computer, to image points of the x-ray image, in order to register the second x-ray image;
      j) via the common coordinate system of the region to be imaged, a transformation matrix for the positional change of the x-ray beam cone (30, 32) between the first and the second image is determined;
      k) the images are oriented and scaled relative to each other using the matrix, wherein a size scaling is determined true to scale from the positional change in the z-direction, a distance between the images is determined true to scale from the positional change in the x- and/or y-direction, a rotation is determined true to scale from an angle $\Omega$, and an inclination of the second image relative to the plane of the first image is determined true to scale from angles $\Theta$ and $\Phi$;

l) the images are oriented relative to each other and displayed using the determined values.

2. The method according to one of the preceding claims, wherein further x-ray images are taken from other movement positions and are joined, in adapted manner and according to the second x-ray image, to the first x-ray image or to a total display generated from the first x-ray image.

3. The method according to one of the preceding claims, wherein a first set of images comprising the first and the second image is generated relative to a first common position, and a second set of images including the first and the second image is generated at a second common position different from the first one, wherein the common positions are rotated relative to each other and/or spaced from each other by an angle.

4. The method according to claim 3, wherein the angle is about 90°.

5. The method according to one of the preceding claims, wherein possible positioning actions are displayed to a user using a display device.

6. The method according to one of the preceding claims, wherein the acquisition arrangement comprises a motor arrangement which implements the desired positioning actions using manual control or automatically.

**Revendications**

1. Procédé de représentation orientée de prises de vue d'une région à représenter, photographiée avec un système de navigation (20, 21, 22) en au moins deux étapes de prise de vue, notamment d'un fémur (28), où les prises de vue sont localisables dans un système de coordonnées commun de la région à représenter, où le système de navigation (20, 21, 22) comporte les caractéristiques suivantes :

- un détecteur de rayons X (34, 34a) avec une source de rayons X (S, S') disposée en regard de celui-ci, aptes à être déplacés l'un par rapport à l'autre et par rapport à la région à représenter selon un rapport pré-déterminable;
- un système de prise de vue stéréoscopique (20), par exemple un appareil stéréoscopique ;
- plusieurs dispositifs de marquage (26a) disposés dans une position fixe sur le détecteur de rayons X (34, 34a) et la région à représenter, et qui sont au moins partiellement détectables par le système de prise de vue stéréoscopique (20) ;

où le procédé est **caractérisé par** les étapes suivantes :

a) une première prise de vue de la région à représenter est déclenchée par l'appareil de radiographie dans une première position,

b) le dispositif de navigation détecte le déclenchement de la prise de vue au moyen d'une analyse d'image et émet un premier signal de commande ;

c) le signal de commande ordonne au système de prise de vue stéréoscopique de détecter des premières positions de la pluralité de dispositifs de marquage (26a) sur le détecteur de rayons X (34) et sur la région à représenter ;

d) des données concernant les premières positions sont enregistrées ;

e) les données concernant les premières positions sont mises en relation à l'aide d'un ordinateur avec des points d'image de la prise de vue radiographique afin d'enregistrer la prise de vue radiographique ;

f) le détecteur de rayons X (34, 34a) et la source de rayons X (S, S') sont déplacés vers une deuxième position relativement à la région à représenter ;

g) une deuxième prise de vue est déclenchée par le détecteur de rayons X (34, 34a), un deuxième signal de commande étant émis ;

h) le deuxième signal de commande ordonne au système de prise de vue stéréoscopique (20) de détecter des deuxièmes positions des dispositifs de marquage (26a) sur le détecteur de rayons X (34, 34a) et sur la région à représenter ;

i) des données concernant les deuxièmes positions sont mises en relation à l'aide d'un ordinateur avec des points d'image de la prise de vue radiographique afin d'enregistrer la deuxième prise de vue radiographique ;

j) une matrice de transformation concernant un changement de position du cône de rayonnement des rayons X (30, 32) entre la première et la deuxième prise de vue est déterminé via le système de coordonnées commun de la région à représenter ;

k) les prises de vue sont orientées et redimensionnées l'une par rapport à l'autre au moyen de la matrice, où un redimensionnement à l'échelle est déterminé à partir du changement de position dans la direction z, un espacement à l'échelle entre les prises de vue est déterminé à partir du changement de position dans la direction x et/ou y, une rotation à l'échelle est déterminée à partir d'un angle $\Omega$ et un basculement à l'échelle de la deuxième prise de vue par rapport au plan de la premier prise de vue est déterminé à partir des angles $\Theta$ et $\Phi$ ;

l) les prises de vue sont orientées au moyen des valeurs déterminées et sont représentées.

2. Procédé selon l'une quelconque des revendications précédentes, où d'autres prises de vue radiographiques sont prises depuis d'autres positions et rattachées, sous une forme adaptée, conformément à la deuxième prise de vue radiographique, à la première prise de vue radiographique ou à une représentation globale générée à partir de celle-ci.

3. Procédé selon l'une quelconque des revendications précédentes, où un premier ensemble de prises de vue comportant la première et la deuxième prise de vue est réalisé par rapport à une première position commune et un deuxième ensemble de prises de vue comportant une autre première et deuxième prise de vue est réalisé dans une deuxième position commune différente de la première, où les positions communes forment un angle l'une par rapport à l'autre et/ou sont à distance l'une de l'autre.

4. Procédé selon la revendication 3, où l'angle est d'environ 90°.

5. Procédé selon l'une quelconque des revendications précédentes, où de potentielles mesures de positionnement sont présentées à un opérateur au moyen d'un dispositif d'affichage.

6. Procédé selon l'une quelconque des revendications précédentes, où le dispositif de prise de vue comporte un dispositif motorisé qui met en oeuvre, manuellement ou automatiquement, les mesures de positionnement souhaitées.

Fig. 1a

Fig. 1b

Fig. 2

Fig. 3a

Fig. 3b

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1782734 A
- US 20030179856 A

- DE 10210645